# EUROPEAN PATENT APPLICATION

(11) **EP 1 167 968 A1**
(43) Date of publication of application: **02.01.2002**
(21) Application number: 00912914.9
(22) Date of filing: 29.03.2000
(51) Int. Cl.: G01N 33/48

(54) **SAMPLE EXAMINING INSTRUMENT AND WIPING-OUT EXAMINING INSTRUMENT**

(30) Priority: 30.03.1999 JP 8937899
(71) Applicant: KIKKOMAN CORPORATION, Noda-shi, Chiba 278-0037 (JP)
(72) Inventor: Igarashi, Toshinori, Noda-shi, Chiba-ken 278-0037 (JP); Murakami, Seiji, 250, Noda, Noda-shi, Chiba 278-0037 (JP); Saito, Morisaku, Tokyo 116-0002 (JP)
(74) Representative: Hague, Alison Jane
(86) International application number: JP0001951
(87) International publication number: WO0060348

(57) **Abstract**

A sample examining instrument comprising a test-tube-form measuring container storing therein a reaction reagent such as an emission reagent, and a reagent container inserted for burying into the upper opening of the measuring container, wherein sealants are pasted to the upper and lower portions of the reagent container and a reagent such as an extract liquid is sealed in the reagent container, whereby providing the sample examining instrument downsized due to the reagent container fitted into the measuring container.

## Description

### TECHNICAL FIELD

This invention relates to an instrument for taking a sample of soil or bacteria from a liquid or a solid surface and reacting it with a reagent to determine the degree of its contamination.

### BACKGROUND ART

International Publication No. WO 97/03209 published under the PCT and entitled "TEST APPARATUS, SYSTEM AND METHOD FOR THE DETECTION OF TEST SAMPLES" shows a known example of apparatus for wiping soil from a solid surface with a swab and reacting it with a reagent to determine the degree of its contamination.

The apparatus includes a test unit. The test unit comprises a tubular body, a swab inserted in the body and a reagent container attached to the lower end of the body. If the swab is forced down to pierce a seal for the reagent container, the wad carried at the end of the swab is brought into contact with the reagent in the container. Thus, it is possible to determine the degree of contamination of the material to be tested.

The swab in the known apparatus has, however, to be stopped during its downward movement for, for example, reaction with an extracting agent. The apparatus does, however, not have any mechanism for stopping the swab, though it has an indicator, but it relies entirely upon the carefulness of its user for the proper stopping of the swab. Moreover, there is no disclosure of any member for breaking the seal. Moreover, there is no mechanism by which the soil collected with the swab can be suspended thoroughly in the reagent, but an error is likely to occur when the degree of contamination is determined. Moreover, the mere end-to-end connection of the body and reagent container reflects the absence of any effort to be made for a reduction in size of the apparatus.

As a result of our research efforts, we, the inventors of this invention, have found that the insertion of a reagent container holding a reagent, such as an extracting agent, in the opening of a measuring container holding a luminescent reagent, such as luciferase, makes a smaller apparatus containing the reagents in the measuring container, or tube, and that a breaker situated at the bottom of the body, or reagent container for breaking a seal ensures the progressive contact between the swab and the extracting agent, and then between the extracting agent and the reagent.

### DISCLOSURE OF THE INVENTION

According to a first aspect of this invention, there is provided a sample testing instrument which comprises a testing container shaped like a test tube and holding a reaction reagent, and a tubular reagent container having a top opening and a bottom opening each closed with a sealing material, and holding a reagent, the testing container having a top opening in which the reagent container is fitted.

The fitting of the reagent container in the testing container enables a reduction in size of the instrument. The reagent container is preferably so fitted in the testing container that its top may be flush with the top of the testing container.

The reagent container has a breaker situated toward the bottom of its interior for breaking the sealing material at its bottom. Moreover, it has another breaker situated above its top for breaking the sealing material at its top.

According to a second aspect of this invention, there is provided a wiping test instrument which comprises a tubular body having a top opening and a bottom opening, a sampling device having a holding member fitted removably in the top opening of the tubular body and a swab held by the holding member, and a sample testing instrument composed of a testing container shaped like a test tube and holding a reaction reagent, and a tubular reagent container having a top opening and a bottom opening each closed with a sealing material, and holding a reagent, the testing container having a top opening in which the reagent container is fitted, the testing instrument being fitted in the bottom opening of the tubular body.

### BRIEF DESCRIPTION OF THE DRAWINGS

FIG. 1 is a longitudinal sectional view of a wiping test instrument according to a first embodiment of this invention;
FIG. 2 is an enlarged view of the sample testing instrument shown in FIG. 1;
FIG. 3 is an enlarged view of the reagent container shown in FIG. 2;
FIG. 4 is a sectional view taken along the line 4-4 in FIG. 3;
FIG. 5 is an enlarged front elevation of a reagent container having a multiplicity of slits formed in its surface;
FIG. 6 is an enlarged sectional view taken along the line 6-6 in FIG. 1;
FIG. 7 is a sectional view showing in detail the relation between slits in a main body and a flange on a holding member in the device shown in FIG. 1;
FIG. 8 is a side elevation of the device shown in FIG. 7, showing a slit;
FIG. 9 is a view similar to FIG. 2, but showing a modified form of device intended for use as an independent sample testing instrument;
FIG. 10 is a view similar to FIG. 9, but showing another modified form of device;
FIG. 11 is a longitudinal sectional view of a wiping test instrument according to a second embodiment of this invention;
FIGS. 12A to 12D are a series of schematic sectional views showing a process for assembling the sample testing instrument shown in FIG. 11;
FIG. 13 is an enlarged sectional view taken along the line 13-13 in FIG. 12C;
FIGS. 14A to 14D are a series of schematic sectional views showing a process for assembling a modified form of the sample testing instrument shown in FIG. 11, in which a scraper having a bottom closed with a sealing material holds an extracting agent; and
FIG. 15 is a longitudinal sectional view of a wiping test instrument according to a third embodiment of this invention.

### BEST MODE FOR CARRYING OUT THE INVENTION

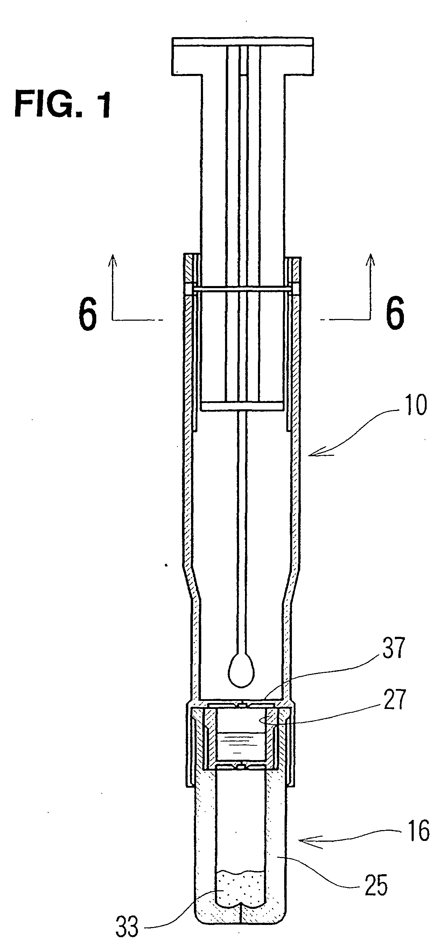

Referring to FIG. 1, a wiping test instrument 10 according to a first embodiment of this invention has a tubular body 12 having a top opening and a bottom opening, a sample wiping, or sampling device 14 fitted removably in the top opening 13 of the body 12 and a sample testing instrument 16 fitted in the bottom opening 15 of the body 12 for examining the sample for bacterial soil.

The sampling device 14 has a swab 19 and a holding member 20 for holding the swab 19. The swab 19 has a rod portion 17 and an egg-shaped wad portion 18 attached to the lower end of the rod portion 17. The sampling device 14 is removable from the tubular body 12 so that the swab 19 may wipe a sample surface to collect bacteria therefrom.

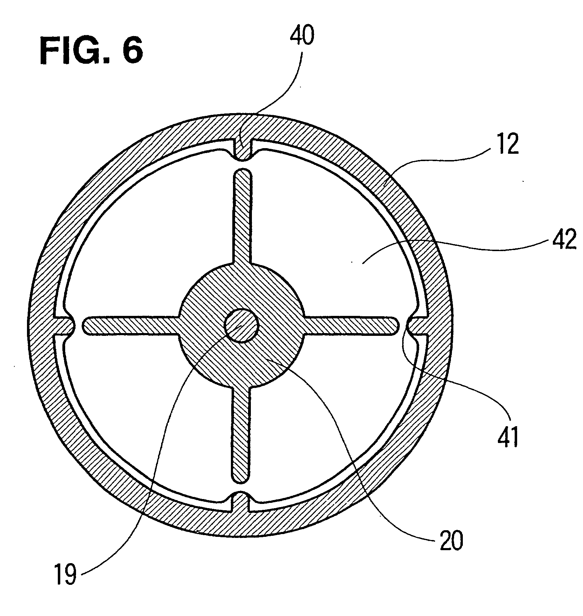

The holding member 20 has a lower portion 21 of small diameter and an upper portion 22 of large diameter. The small-diameter portion 21 has such a diameter that the holding member 20 may stop in any position when inserted in the tubular body 12. The small- and large-diameter portions 21 and 22 define therebetween a laterally projecting surface 23 serving as a stop for preventing any further downward movement of the sample wiping device 14 by resting on the top end 24 of the tubular body 12. The tubular body 12 has a plurality of straight and longitudinally extending protrusions 40 formed on the inner wall surface of its upper portion, as shown in FIG. 6. The holding member 20 has in its middle portion a flange 42 having an outer edge contacting the top edges 41 of the protrusions 40, so that the flange 42 may have only a small area of contact with the tubular body 12 and thereby enable the holding member 20 to move smoothly relative to the tubular body 12.

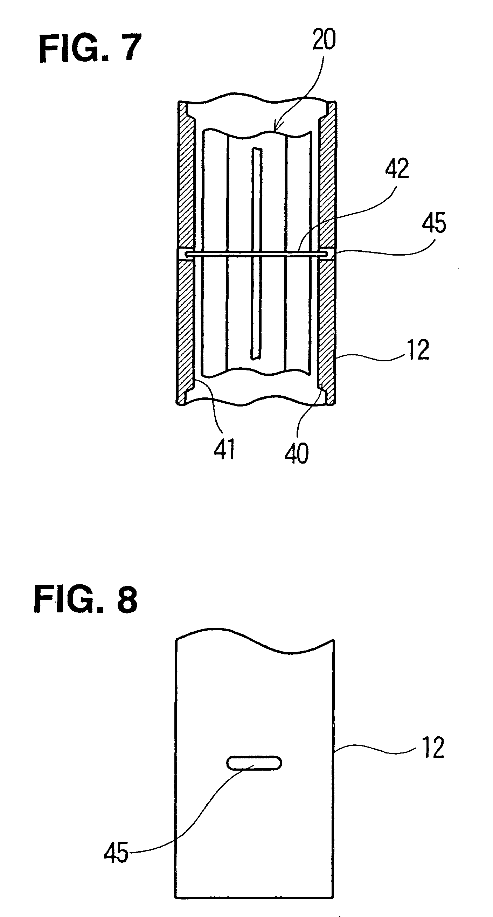

The tubular body 12 preferably has slits 45 with which the flange 42 is engageable, as shown in FIGS. 7 and 8, so that the holding member 20 may be held in its intermediate position relative to the tubular body 12, as shown in FIG. 1, when the instrument 10 is shipped as a product.

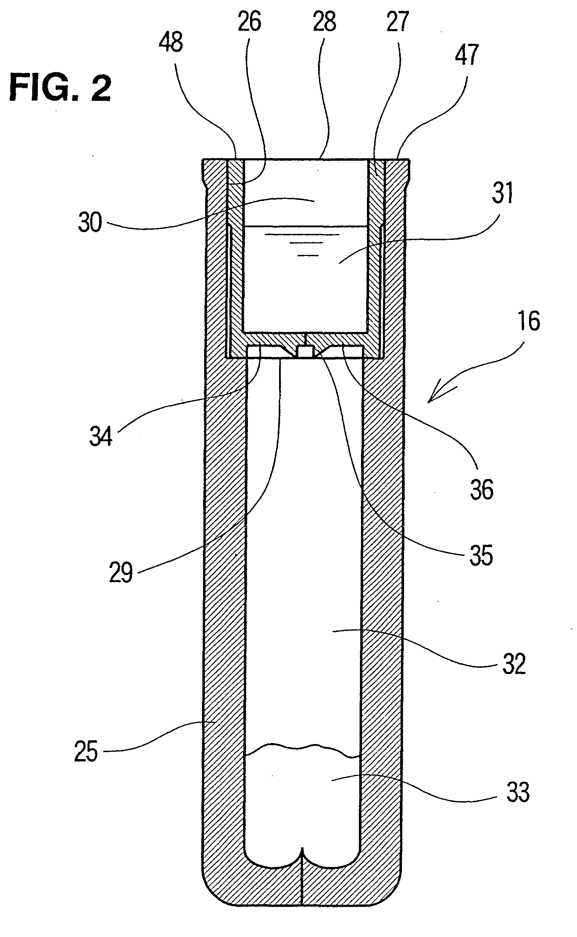

FIG. 2 shows the sample testing instrument 16. The instrument 16 has a measuring container 25 shaped like a test tube and a reagent container 27 fitted in the top opening 26 of the measuring container 25. The instrument 16 is so fitted in the bottom opening 15 of the tubular body 12 as to engage a shoulder 43 formed above the top opening 15, as shown in FIG. 1.

The reagent container 27 is cylindrical, and has a tightly closed chamber 30 defined by a sealing material 28 and a sealing material 29 closing its top and bottom openings, respectively. The chamber 30 holds an extracting agent 31 for separating bacteria from the wad portion 18. The reagent container 27 fitted in the measuring container 25 defines a tightly closed chamber 32 therein. The chamber 32 holds a reaction reagent 33.

The reagent container 27 fitted in the measuring container 25 has a top end 48 which is flush with its top end 47, so that the instrument 16 may have only an overall length equal to the length of the measuring container 25 and be accordingly small in size. The sealing material 28 extends over the top end 47 of the measuring container 25 to seal the opening of the instrument 16 completely. Thus, the instrument 16 is a device holding two kinds of reagents and not admitting any germ during its sale as an independent article.

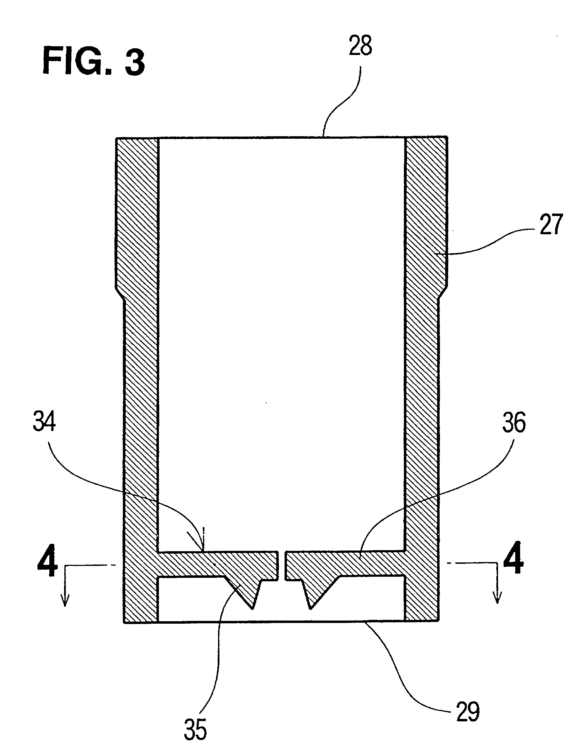

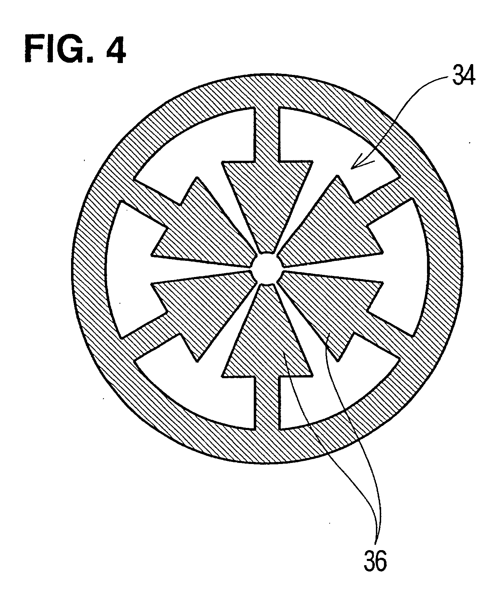

The reagent container 27 has a breaker 34 near its bottom, as shown in FIG. 3. The breaker 34 is formed by, for example, six arrow-shaped breaker members 36 extending radially inwardly from the inner wall surface of the reagent container 27, as shown in FIG. 4. Each breaker member 36 has a downward projection 35 formed near its free end. If the swab 19 is forced down, the breaker members 36 are bent downwardly so that the projections 35 may break the sealing material 29. The shoulder 43 in the tubular body 12 has a breaker 37 which is similar in construction to the breaker 34 in the reagent container 27, as shown in FIG. 1. The breaker 37 is caused by the downward movement of the swab 19 to break the sealing material 28, as the breaker 34 does.

The breakers 34 and 37 are not essential if the sealing materials 28 and 29 are easy to break with the swab 19.

The breaker 34 has the function of scraping the surface of the wad portion 18 of the swab 19 and thereby promoting the separation of bacteria from the surface of the wad portion 18, as shown in FIG. 1. Although the downward movement of the swab 19 may be somewhat resisted by the breaker 34, its resistance prolongs the contact of the wad portion 18 with the extracting agent 31. It is also possible to stop the downward movement of the swab 19 upon its arrival at the extracting agent 31.

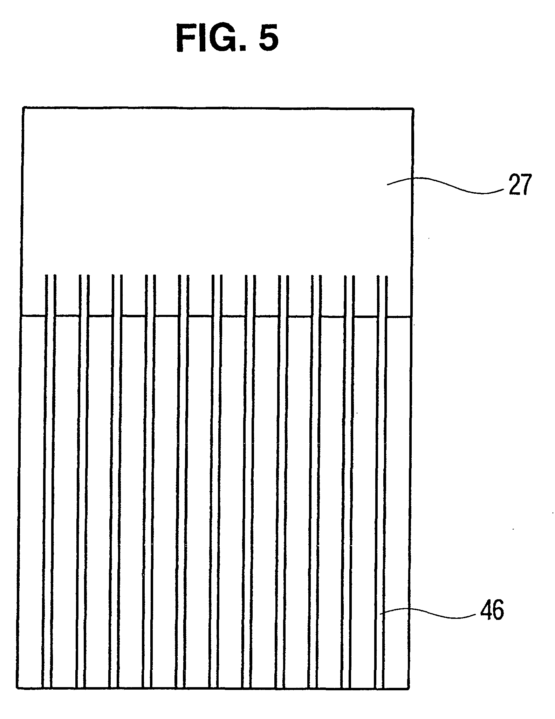

The reagent container 27 has a multiplicity of straight grooves 46 formed in its outer wall surface, as shown in FIG. 5. The grooves 46 serve as air passages for preventing the compression of the air in the measuring container 25 when the reagent container 27 is fitted therein, as shown in FIG. 2. The grooves 46 serve as air passages during the freeze drying of the reaction reagent 33, too.

While this invention is basically a device for detecting bacteria by employing a luminescent reaction, it is not limited thereto, but is also useful for the collection, culture and inspection of microorganisms.

A cationic surface active agent can, for example, be used as the extracting agent for a luminescent reaction.

The reaction reagent may be a composition containing luciferase, luciferin, magnesium ions, dithiothreitol and a buffer solution, or containing luciferase, dithiothreitol, pyruvate orthophosphate diquinase, phosphoenolpyruvic acid, sodium pyrophosphate, luciferin, magnesium ions and a buffer solution.

The collection, culture and inspection of microorganisms may be carried out by using a culture medium, or an inspection reagent instead of the extracting agent.

Description will now be made of the operation of the instrument 10 according to the first embodiment of this invention.

The sampling device 14 is supplied as partly inserted in the tubular body 12, as shown in FIG. 1. The extracting agent 31 is held in the reagent container 27, and the reaction reagent 33 in the measuring container 25.

Then, the sampling device 14 is pulled out of the tubular body 12 and the swab 19 is rubbed against a sample surface to collect bacteria therefrom. The device 14 is inserted into the tubular body 12 again. The device 14, or the swab 19 is forced down to cause the breaker 37 to break the sealing material 28 so that the wad portion 18 may be dipped in the extracting agent 31.

When the swab 19 is further forced down for dipping its wad portion 18 in the extracting agent 31, it is resisted by the breaker 34 and it necessarily follows that the wad portion 18 remains in contact with the extracting agent 31 for a prolonged time. This ensures the migration of bacteria from the swab 19 into the extracting agent 31. When the breaker 34 is opened like a flower by the swab 19, it scrapes the surface of the swab 19 with the free ends of its members and thereby ensures the efficient transfer of bacteria from the wad portion 18 into the extracting agent 31.

Then, the sampling device 14 is pushed down until the laterally projecting surface 23 of the holding member 20 abuts on the top end 24 of the tubular body 12. As a result, the sealing material 29 is broken by the members 36 of the breaker 34 to allow the extracting agent 31 containing bacteria to drop into the measuring container 25.

Finally, the whole instrument 10 is slightly shaken to promote the contact of the extracting agent 31 with the reaction reagent 33 so that bacteria may be detected by a luminescent reaction.

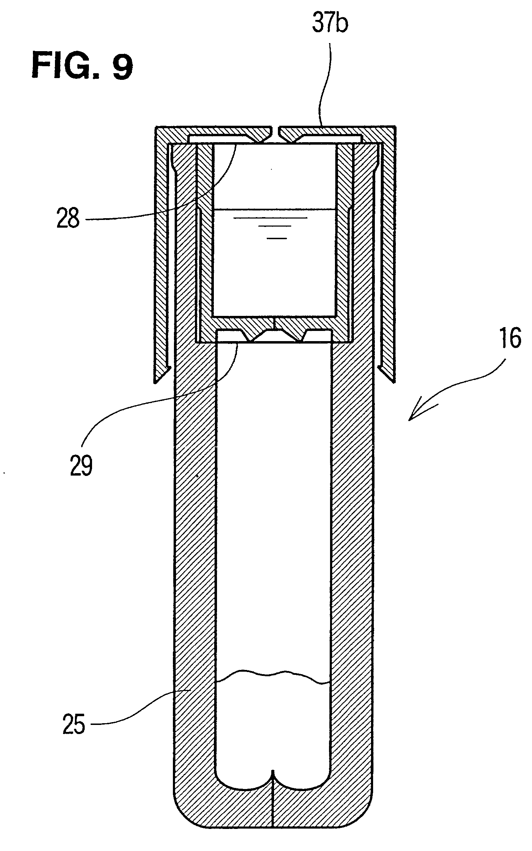

While the foregoing description has been of the wiping test instrument 10 according to the first embodiment of this invention, the sample testing instrument 16 shown in FIG. 2 can be used as an independent testing device, and moreover, FIG. 9 shows a modified form thereof having a breaker 37b fitted over the sealing material 28 for breaking it. A swab, which may be a commercially available product, is rubbed against a sample, and is inserted into the top of the instrument 16 for breaking the sealingmaterials 28 and 29. The operation of the apparatus according to the first embodiment is thereafter repeated for detecting the soil of the sample.

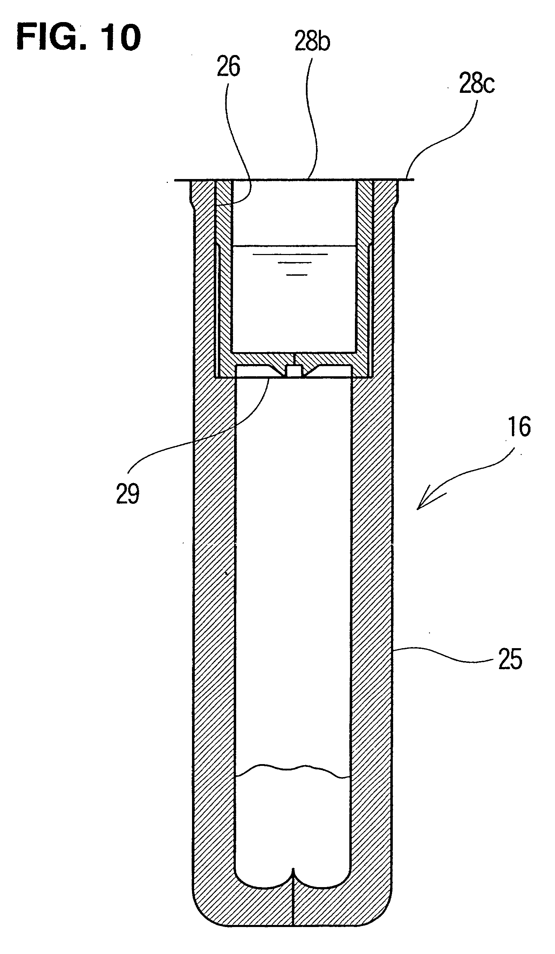

FIG. 10 shows another modified form of the sample testing instrument 16 that can be used as an independent device. The modified instrument 16 is a modification to the instrument 16 shown in FIG. 2 and includes a measuring container 25 having an opening 26 sealed with a sealing material 28b having a diameter which is larger than the outside diameter of the container 25 at its opening 26. The sealing material 28b has a portion 28c projecting radially outwardly from the container 25, and the projecting portion 28c can be gripped between fingers to remove the sealing material 28b, so that the sealing material 28b may not have to be broken with a swab 19. The instrument 16 of the construction as described allows the use of a strong sealing material and thereby provides an independent product of still improved quality.

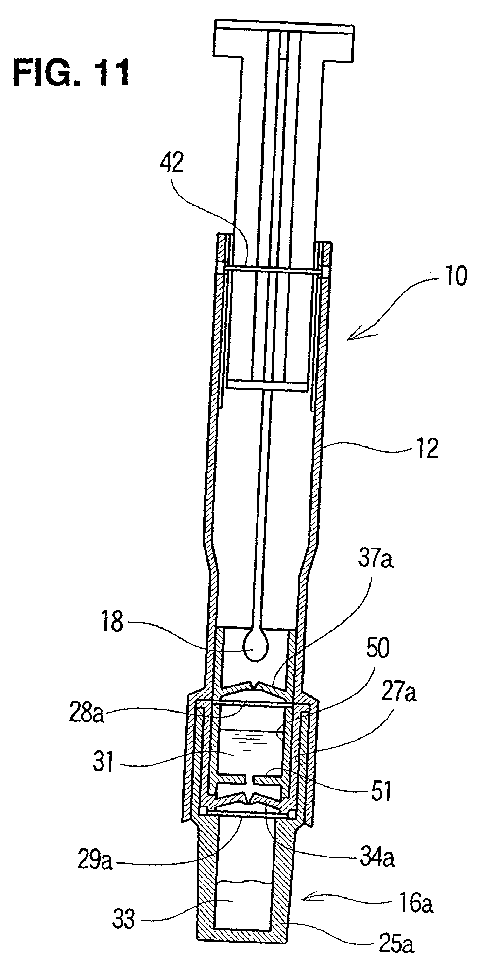

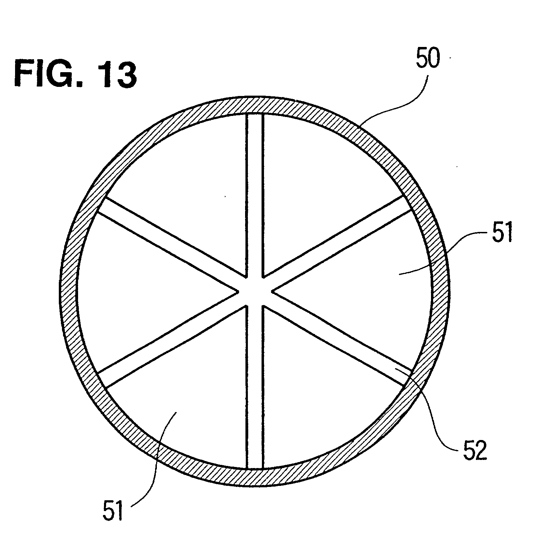

FIG. 11 shows a sample testing instrument according to a second embodiment of this invention. The instrument 16a has a measuring container 25a and a reagent container 27a fitted in the measuring container 25a and holding an extracting agent 31. The reagent container 27a is cylindrical and has at its bottom a breaker 34a which is similar in construction to the breaker 34 shown in FIG. 2. A scraper 50 for scraping the surface of a wad portion 18 is fitted in the reagent container 27a. The scraper 50 is cylindrical and has a plurality of scraping members 51 at its bottom. The scraper has six scraping members 51 as shown by way of example in FIG. 13. The scraping members 51 scrape the surface of the wad portion 18 to promote the separation of bacteria therefrom. The reagent container 27a has its bottom closed with a sealing material 29a and the scraper 50 has a top closed with a sealing material 28a. The measuring container 25a holds a reaction reagent 33. The scraper 50 holds the extracting agent 31. A breaker 37a is similar in construction to the breaker 34a and is carried by a tubular body 12 above the sealing material 28a.

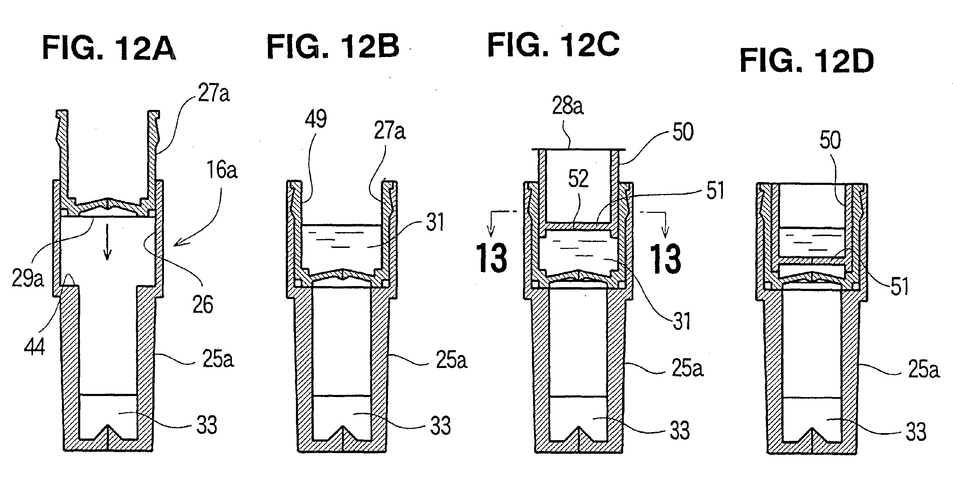

A process for assembling the instrument 16a as shown in FIG. 11 will now be described with reference to FIGS. 12A to 12D. First, the reagent container 27a having the sealing material 29a attached to its bottom is pressed into the measuring container 25a, which may, for example, hold the reaction reagent 33 in a freeze-dried form, through its top opening 26 until the container 27a rests on a shoulder 44 formed in the container 25a (see FIG. 12A).

Then, the extracting agent 31 is poured into the reagent container 27a, as shown in FIG. 12B. Finally, the scraper 50 carrying the sealing material 28a is pressed into the opening 49 of the reagent container 27a, as shown in FIG. 12C. As a result, the extracting agent 31 flows into the scraper 50 through the clearance 52 between every two adjoining scraping members 51 (see FIG. 13). Thus, the extracting agent 31 is held in the scraper 50, and the reaction reagent 33 in the measuring container 25a.

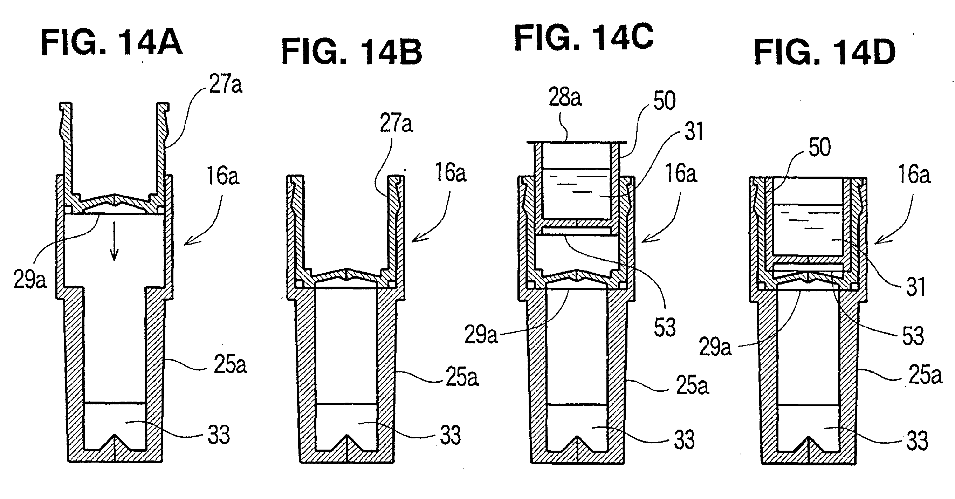

FIGS. 14A to 14D show a process for assembling a modified form of instrument 16a according to the second embodiment of this invention as shown in FIG. 11. The modified instrument has a scraper 50 having a top opening closed with a sealing material 28a and a bottom opening closed with a sealing material 53, as shown in FIG. 14D. Thus, the extracting agent 31 is held in the scraper 50 by the top and bottom sealing materials 28a and 53. The scraper 50 holding the extracting agent 31 can be prepared as an independent article for use in the process for assembling the instrument 16a as shown in FIGS. 14A to 14D.

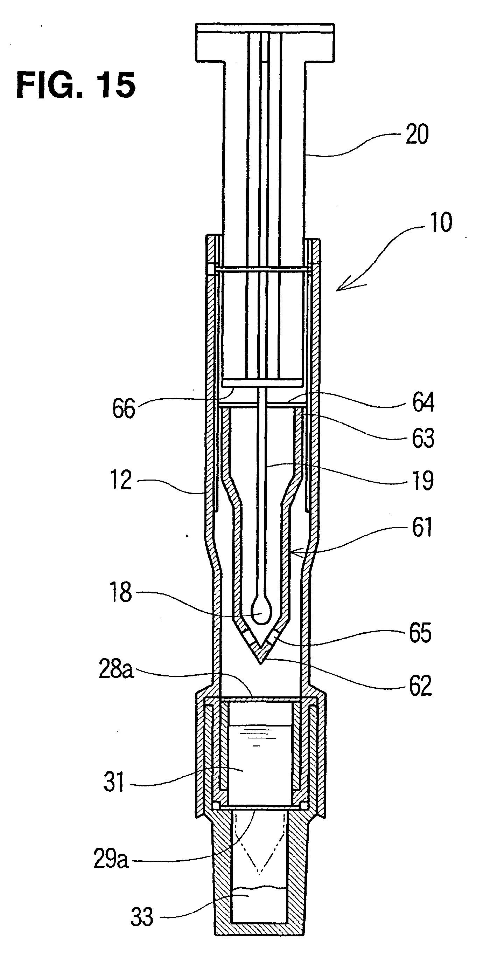

FIG. 15 shows a wiping test instrument 10 according to a third embodiment of this invention which includes a movable breaker. The breaker 61 is cylindrical and has a sharp end 62. The breaker 61 has a flange 64 at its top or base end 63. The flange 64 is slidable on the inner wall surface of a tubular body 12 to permit the vertical movement of the breaker 61 along the tubular body 12. The breaker 61 has a plurality of ports 65 formed adjacent to its bottom end 62. The breaker 61 stays in the middle portion of the tubular body 12. The breaker 61 contains a swab 19.

Description will now be made of the operation of the breaker 61. The breaker 61 is moved down with a holding member 20 as its base end 63 is pressed by the bottom end 66 of the holding member 20. The breaker 61 breaks a sealing material 28a and then a sealing material 29a to enable the contact between the wad portion 18 of the swab and an extracting agent 31, and then between the extracting agent 31 and a reaction reagent 33. The contact between the wad portion 18 and the extracting agent 31 is possible owing to the presence of the ports 65. The vertically movable breaker 61 ensures the breaking of the sealing materials.

### INDUSTRIAL APPLICABILITY

The wiping test instrument of this invention is small enough for the convenience of transportation and is suitable for use as a testing device for extracting easily and effectively bacteria collected with a swab.

## Claims

1. A sample testing instrument comprising:
a testing container shaped like a test tube and holding a reaction reagent; and
a tubular reagent container having a top opening and a bottom opening each closed with a sealing material, and holding a reagent;
the testing container having a top opening in which the reagent container is fitted.

2. The instrument according to claim 1, further including a breaker situated in the reagent container near its bottom for breaking the sealing material closing its bottom opening.

3. The instrument according to claim 1, further including a breaker situated above the top of the reagent container for breaking the sealing material above it.

4. The instrument according to claim 1, wherein the reagent container has a top which is flush with the top of the testing container.

5. A wiping test instrument comprising:
a tubular body having a top opening and a bottom opening;
a sampling device having a holding member fitted removably in the top opening of the tubular body and a swab held by the holding member; and
a sample testing instrument composed of a testing container shaped like a test tube and holding a reaction reagent, and a tubular reagent container having a top opening and a bottom opening each closed with a sealing material, and holding a reagent, the testing container having a top opening in which the reagent container is fitted, the testing instrument being fitted in the bottom opening of the tubular body.

6. The instrument according to claim 5, further including a breaker situated in the tubular body near its bottom for breaking the sealing material.

7. The instrument according to claim 5, further including a breaker situated in the reagent container near its bottom for breaking the sealing material.

8. The instrument according to claim 5, wherein the reagent container has a top which is flush with the top of the testing container.
